# EUROPEAN PATENT SPECIFICATION

(11) **EP 3 903 743 B1**
(45) Date of publication and mention of the grant of the patent: **27.03.2024**
(21) Application number: 19906127.6
(22) Date of filing: 24.12.2019
(51) Int. Cl.: A61F 2/95

(54) **STENT DELIVERY DEVICE**
VORRICHTUNG ZUM ZUFÜHREN EINES STENTS
DISPOSITIF DE TRANSPORT DE STENT

(30) Priority: 28.12.2018 CN 201811625327; 28.12.2018 CN 201822243522 U
(43) Date of publication of application: 03.11.2021
(73) Proprietor: Hangzhou Endonom Medtech Co., Ltd, Hangzhou, Zhejiang 310051 (CN)
(72) Inventor: LI, Jianmin, Hangzhou, Zhejiang 310051 (CN); WANG, Yongsheng, Hangzhou, Zhejiang 310051 (CN)
(74) Representative: HWP Intellectual Property
(86) International application number: PCT/CN2019/128095
(87) International publication number: WO 2020/135455

(56) References cited:
- US-A1- 2008 082 158
- US-A1- 2008 132 989
- US-A1- 2014 257 454

## Description

### TECHNICAL FIELD

The present application relates to the technical field of medical instruments, and more particularly to a stent delivery device for releasing a stent into a vessel.

### BACKGROUND

Aortic diseases, such as aortic aneurysm and aortic dissection, are one kind of the most pernicious and hardest vascular surgical diseases to treat. Traditional treatment methods, such as laparotomy and prosthetic vessel replacement, have the risks of severe surgical trauma and high fatality rate. In recent years, a minimally invasive and simple interventional operation method has been developed, in which a covered stent is implanted in a lesion site of a vessel, and the covered stent conforms to the inner wall of the vessel to isolate the lesion site of the vessel from the blood flow. The covered stent can not only allow the blood to flow through normally, but also protect the lesion site of the vessel and effectively repair the lesion site of the vessel.
For example, US 2008082158A1 discloses a method for deployment of a stent graft that includes providing a delivery device. The delivery device includes a support stent, an inner sheath, and an outer sheath. The support stent is slidably positioned about a wire lumen. The support stent has a proximal end and a distal end and is expandable from a compressed delivery configuration to an expanded configuration. The inner sheath is retractably positioned about the support stent with the support stent in the delivery configuration. The anchor stent is slidably positioned about the inner sheath and is deployable from a compressed delivery configuration to a deployed configuration. The outer sheath is retracted to expand the anchor stent from the delivery configuration to an expanded configuration. The anchor stent is deployed in the body vessel. The inner sheath is retracted to expand the support stent from the compressed insertion configuration to an expanded configuration. For another example, US2008/132989 A1 discloses devices and methods for controlling expandable prosthesis during deployment. A radially expandable control member is positionable within the prostheses and has an expanded shape which engages an inner surface of the prostheses to urge the prostheses outwardly against the sheath. The radially expandable control member therefore controls axial position of the prostheses during deployment. Thus one or more prostheses may be deployed at a treatment site precisely. When multiple prostheses are deployed, excessive spacing or overlap between adjacent prostheses is minimized. The prostheses of the present invention are often deployed in stenotic lesions in peripheral arteries as well as coronary arteries and other body lumens.
However, how to provide a stent delivery device capable of effectively releasing the stent into the lesion site of the vessel to improve the effect of treating vascular diseases has become a technical problem to be solved.

### SUMMARY

The present application provides a stent delivery device according to independent claim 1 capable of effectively releasing a stent into a lesion site of a vessel.

The stent delivery device provided in the present application is configured to release the stent into the vessel, and includes:
a fixing assembly, including a fixing member and a limiting member, wherein one end of the limiting member passes through the stent and abuts against the fixing member so as to lock the stent; and
a releasing assembly, including a movable member and a locking member connected to the movable member, wherein the movable member is connected to the other end of the limiting member,
wherein an outer peripheral surface of the movable member is provided with a first sliding groove and a second sliding groove communicating end to end, the first sliding groove extends along a circumferential direction, the second sliding groove extends along an axial direction, and one end of the locking member is capable of sliding in the first sliding groove and the second sliding groove;
the locking member is slidably connected to the movable member such that the locking member is slidable relative to the movable member along the first sliding groove in the circumferential direction between a first and a second position,
when the locking member is in the first position, the movable member is fixed relative to the fixing member along an axial direction under the restriction of the locking member, and
when the locking member slides to the second position, the movable member is capable of driving the limiting member to move along with it away from the fixing member along the axial direction so as to release the stent.

The locking member is provided to lock the movable member so as to prevent the movable member from driving the limiting member to release the stent when there is no need to release the stent. The locking member is provided to move to different positions so as to control the locking member to lock or release the movable member. Since the position of the locking member is variable, it may be rapidly determined whether the movable member is in a fastened state or a released state. Further, when the movable member is in the released state, the limiting member may be driven to lock or release the stent, so as to speed up the release of the stent and save the surgical treatment time.

### BRIEF DESCRIPTION OF THE DRAWINGS

In order to illustrate the technical solutions of the embodiments according to the present application more clearly, drawings used in the description of the embodiments according to the present application will be briefly introduced below. It should be appreciated that the drawings described below merely illustrate some embodiments of the present application, and other drawings may be obtained by those skilled in the art without departing from the scope of the drawings.
FIG. 1 is a schematic diagram showing a structure of a stent delivery device according to an embodiment of the present application.
FIG. 2 is a cross-sectional view showing a stent delivery device according to an embodiment of the present application.
FIG. 3 is a partial cross-sectional view showing a proximal end of a stent delivery device according to an embodiment of the present application.
FIG. 4 is a partial cross-sectional view showing a distal end of a stent delivery device according to an embodiment of the present application.
FIG. 5 is a schematic diagram showing a structure of a distal end of a stent delivery device according to an embodiment of the present application.
FIG. 6 is an exploded view of a releasing assembly in a stent delivery device according to an embodiment of the present application.
FIG. 7 is a partial perspective view showing a proximal end of a stent delivery device according to an embodiment of the present application.
FIG. 8 is a cross-sectional view showing an outer sheath sliding assembly in a stent delivery device according to an embodiment of the present application.
FIG. 9 is a partial enlarged cross-sectional view showing a switch in an outer sheath sliding assembly of a stent delivery device according to an embodiment of the present application.

### DESCRIPTION OF THE EMBODIMENTS

The technical solutions of the embodiments of the present application will be clearly and fully described below in combination with accompanying drawings in the embodiments of the present application. For ease of description, "proximal end", "distal end" and "first direction" are involved, wherein the term "proximal end" refers to one end away from an operating end of the stent delivery device, and the term "distal end" refers to one end close to the operating end of the stent delivery device, and the term "first direction" refers to an extending direction of the stent delivery device.

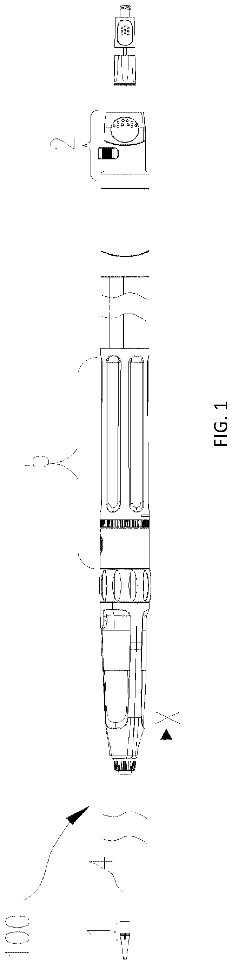

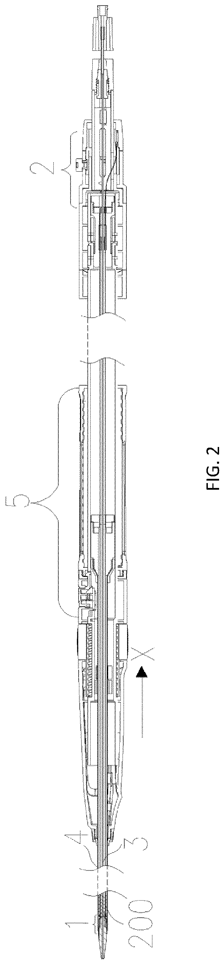

Referring to FIG. 1 and FIG. 2, FIG. 1 shows a stent delivery device 100 according to an embodiment of the present application. The stent delivery device 100 is configured to release the stent 200 into a lesion site of a vessel. The stent 200 includes a tubular rigid wire frame and a polymer membrane fixed on an outer peripheral surface of the tubular rigid wire frame. The tubular rigid wire frame may be obtained by forming an elastic rigid wire bended in a Z-shaped manner into a plurality of rings, and the stent 200 with a membrane is formed by stitching or bonding the plurality of rings with the polymer membrane. During use, the stent 200 is loaded into the stent delivery device 100 after being compressed about its axial axis, and delivered to the lesion site of the vessel by the stent delivery device 100 and then released. Due to the elasticity of the tubular rigid wire frame, the stent 200 automatically returns to its original shape and closely conforms to an inner wall of the vessel to isolate the lesion site of the vessel from the blood flow, thereby achieving the treatment.

With reference to FIG. 1 and FIG. 2, the stent delivery device 100 includes a fixing assembly 1 and a releasing assembly 2. The releasing assembly 2 and the fixing assembly 1 are arranged along a first direction X. The fixing assembly 1 is located at a proximal end of the stent delivery device 100, and the releasing assembly 2 is located at a distal end of the stent delivery device 100, wherein the fixing assembly 1 is configured to secure the stent 200, and the releasing assembly 2 is configured to manipulate the fixing assembly 1 so as to release the stent 200.

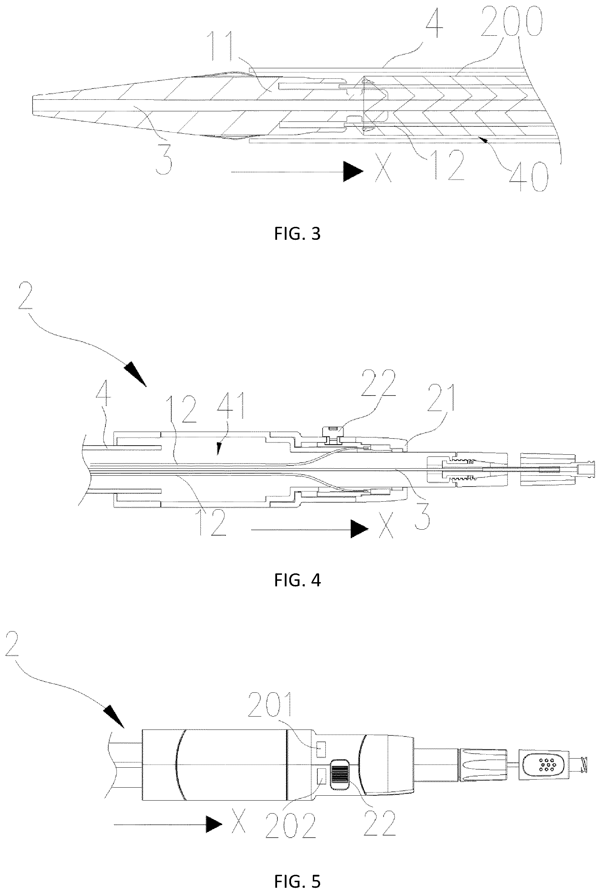

With reference to FIG. 3, the fixing assembly 1 includes a fixing member 11 and a limiting member 12. One end of the limiting member 12 extends through the stent 200 and is slidably connected to the fixing member 11 to lock the stent 200.

Particularly, with reference to FIG. 3, both the stent 200 and the limiting member 12 extend along the first direction X. The proximal end of the stent 200 is provided with a hollowing portion. One end of the limiting member 12 (a proximal end of the limiting member 12) extends through the hollowing portion of the stent 200 and is slidably connected to the fixing member 11, so that the proximal end of the stent 200 is tightened and fixed. In other words, the stent 200 is mounted around the limiting member 12. The distal end of the stent 200 is adjacent to an operating end of the stent delivery device 100, and the distal end of the stent 200 is tightened and restrained by other position of the stent delivery device 100, such that the stent 200 is wholly tightened and restrained to the stent delivery device 100, which brings the convenience for delivering the stent 200 into the vessel with the stent delivery device 100.

With reference to FIG. 4, the releasing assembly 2 includes a movable member 21 and a locking member 22 connected to the movable member 21. The movable member 21 is connected to the other end of the limiting member 12 (a distal end of the limiting member 12). The movable member 21 is configured to drive the limiting member 12 to move away from or close to the fixing member 11 in the first direction X, so that the stent 200 is released or locked by the limiting member 12. The locking member 22 is configured to lock the movable member 21, to prevent the movable member 21 from moving in the first direction X, and thus prevent the limiting member 12 from releasing the stent 200 due to unintentional touch and other reasons when there is no need to release the stent 200 which results in an incorrect position where the stent 200 is released and further incapability of treating the vascular diseases.

With reference to FIG. 4, the locking member 22 is slidably connected to the movable member 21. When the locking member 22 is located at a first position, the movable member 21 is fixed relative to the fixing member 11 under the restriction of the locking member 22. In other words, when the locking member 22 is in the first position, the movable member 21 is locked, so that the movable member 21 cannot drive the limiting member 12 to move in the first direction X. At that time, the limiting member 12 is connected to the fixing member 11. When the locking member 22 slides to a second position, the movable member 21 can drive the limiting member 12 to move away from the fixing member 11 to release the stent 200. In other words, when the locking member 22 is located at the second position, the movable member 21 can drive the limiting member 12 to move in the first direction X, so that the limiting member 12 can moves out from the fixing member. When the limiting member 12 moves to a certain position, it is disengaged from the proximal end of the stent 200. As a result, the stent 200 can be released into the vessel.

The locking member 22 is provided to lock the movable member 21, so as to prevent the movable member 21 from driving the limiting member 12 to release the stent 200 when there is no need to release the stent 200. The locking member 22 is configured such that it can move to different positions, to fasten or release the movable member 21 by controlling the locking member 22. Since the position of the locking member 22 is changeable, it may be rapidly determined that the movable member 21 is in a locked state or a released state. Further, when the movable member 21 is in the released state, the limiting member 12 may be driven to lock or release the stent 200, to speed up the release of the stent 200 and save the surgical treatment time.

In a possible embodiment, with reference to FIG. 5, an outer surface of the releasing assembly 2 is provided with a first marker 201. When the locking member 22 is located at the first position, the locking member 22 is aligned with the first marker 201, and the locking member 22 locks the movable member 21 and the limiting member 12 locks the stent 200 if the stent 200 is provided on the limiting member 12. When the locking member 22 is located at the second position, the locking member 22 is offset from the first marker 201, the movable member 21 is released by the locking member 22, and the movable member 21 is capable of driving the limiting member 12 to move to release the stent 200 therefrom.

Particularly, the first marker 201 corresponds to a state in which the locking member 22 locks the movable member 21. The first marker 201 may be a protrusion or groove provided on the outer surface of the releasing assembly 2, or a marker patch or the like attached to the outer surface of the releasing assembly 2. Certainly, in other embodiments, the first marker 201 may be a status indicator lamp, a buzzer, or the like.

Due to the first marker 201 provided on the outer surface of the releasing assembly 2, which is readily visible by an operator, it is convenient for the operator to quickly and directly determine whether the movable member 21 is in the locked state or the released state. Accordingly, the intuitiveness of determination is improved, the operation is simplified, and the time is saved.

Further, with reference to FIG. 5, the outer surface of the releasing assembly 2 is further provided with a second marker 202 spaced apart from the first marker 201. When the locking member 22 is located at the second position, the locking member 22 is aligned with the second marker 202.

Particularly, the first marker 201 and the second marker 202 may be spaced apart and provided on an outer peripheral surface of the releasing assembly 2. The first marker 201 is adjacent to and aligned with the first position, and the second marker 202 is adjacent to and aligned with the second position.

Particularly, the second marker 202 corresponds to a state in which the fastening member 22 releases the movable member 21. Similarly, the second marker 202 may be a protrusion or groove provided on the outer surface of the releasing assembly 2, or a marker patch attached to the outer surface of the releasing assembly 2. It may further be a state indicator lamp, a buzzer, or the like.

Due to the first marker 201 and the second marker 202 provided on the outer surface of the releasing assembly 2, which are readily visible by the operator, it is convenient for the operator to quickly and directly determine whether the movable member 21 is in the locked state or the released state. Accordingly, the intuitiveness of determination is improved, the operation is simplified, and the time is saved.

With reference to FIG. 2, the movable member 21 has a central axis. The center axis runs along the direction X. The fixing member 11, the limiting member 12 and the movable member 21 are arranged along an extending direction of the central axis.

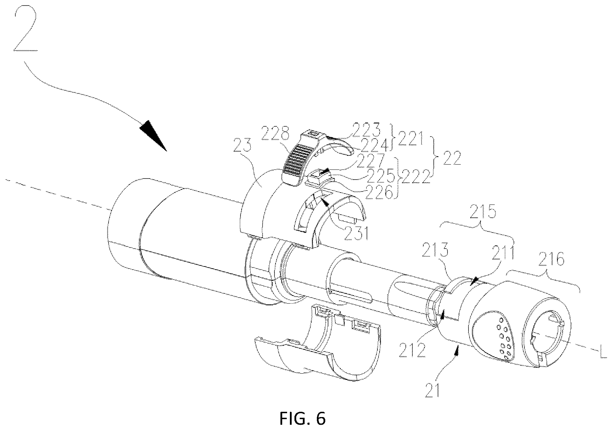

In a possible embodiment, with reference to FIG. 6, an outer peripheral surface of the movable member 21 is provided with a first sliding groove 211 and a second sliding groove 212 communicating end to end. The first sliding groove 211 extends along a circumferential direction of the movable member 21. The second sliding groove 212 extends along the central axis L. One end of the locking member 22 can slide along the first sliding groove 211 and the second sliding groove 212.

Particularly, the movable member 21 may be of a cylindrical shape, with an axial direction thereof as the first direction X. Certainly, in other embodiments, the movable member 21 may also be of a square tubular shape or other tubular shape.

Due to the first sliding groove 211 and the second sliding groove 212 provided in the outer circumferential surface of the movable member 21, with the first sliding groove 211 extending along the circumferential direction of the movable member 21, and the second sliding groove 212 extending along the first direction X, when the locking member 22 is located in the first sliding groove 211, the locking member 22 locks the movable member 21 in the first direction X to prevent the movable member 21 from moving in the first direction X, and the limiting member 12 abuts against the fixing member 11. If the limiting member 12 extends through the stent 200, the limiting member 12 fixes the stent 200 on the stent delivery device 100. When the locking member 22 slides to the second sliding groove 212, the locking member 22 releases the movable member 21, so that the movable member 21 is capable of moving in the first direction X, and in turn the movable member 21 is capable of driving the limiting member 12 to move away from the fixing member 11 in the first direction X, so as to release the stent 200 on the limiting member 12, or the movable member 21 is capable of driving the limiting member 12 to move close to the fixing member 11 along the first direction X, so as to make the limiting member 12 return to an initial position after the stent 200 is released.

Further, the first position and the second position are located at the first sliding groove 211 and the second sliding groove 212, respectively. Particularly, the first position may be any position in the first sliding groove 211, and the second position may be any position in the second sliding groove 212. That is, when the locking member 22 is located in the first sliding groove 211, the movable member 21 is fixed relative to the fixing member 11 under the restriction of the locking member 22. When the locking member 22 slides to the second sliding groove 212, the movable member 21 is capable of driving the limiting member 12 to move away from the fixing member 11 so as to release the stent 200.

Further, with reference to FIG. 6, the movable member 21 has a first end surface 213, through which the second sliding groove 212 extends. When the movable member 21 moves from the proximal end to the distal end along the direction X and moves away from the fixing member 11, the locking member 22 is capable of sliding out of the second sliding groove 212 until it is disengaged from the movable member 21. It should be understood that the first end surface 213 is adjacent to the fixing member 11.

Due to the second sliding groove 212 extending through the first end surface 213, the locking member 22 is capable of sliding out of the second sliding groove 212, and the movable member 21 is then disengaged from the locking member 22. The movable member 21 is controlled to drive the limiting member 12 along the first direction X, so that the limiting member 12 may be quickly separated from the fixing member 11 so as to quickly release the stent 200. Therefore, the stent 200 is released at an accurate position and the surgical time is reduced. Moreover, the movable member 21 disengaged from the locking member 22 is capable of moving freely along an appropriate course, to drive the limiting member 12 to move an appropriate distance, such that the limiting member 12 is capable of releasing the stent 200 without moving too far from the fixing member 11 which results in difficulty of returning to the initial position.

With reference to FIG. 6, the releasing assembly 2 further includes a first sleeve 23. The first sleeve 23 is mounted around a periphery of the movable member 21. The first sleeve 23 is provided with a limiting hole 231. The locking member 22 is arranged on the first sleeve 23. The locking member 22 is slidably connected to the movable member 21 through the limiting hole 231.

It should be understood that the central axis L of the first sleeve 23 runs along the first direction X. The first sleeve 23 can restrict the locking member 22 in a radial direction, so that the locking member 22 may be slidably connected to the movable member 21.

In a possible embodiment, the limiting hole 231 is an elongated hole. Particularly, the limiting hole 231 may extend along a circumferential direction of the first sleeve 23. When the locking member 22 slides, the first sleeve 23 is fixed relative to the fixing member 11, and the locking member 22 can slide in the limiting hole 231. In other embodiments, the locking member 22 may be fixed on the first sleeve 23. When the locking member 22 slides, the locking member 22 and the first sleeve 23 rotate together around the first direction X, to allow one end of the locking member 22 to be slidably connected to the movable member 21.

Further, with reference to FIG. 6, the limiting hole 231 extends along the circumferential direction of the first sleeve 23. The locking member 22 can slide from one end of the first sliding groove 211 to the other end of the first sliding groove 211 along the limiting hole 231. An inner wall of the limiting hole 231 restricts the locking member 22 to be fixed relative to the first sleeve 23 in the direction of the central axis.

The limiting hole 231 which is configured as an elongated hole extending in the circumferential direction provides a sliding space for the locking member 22, so that the locking member 22 is capable of sliding along the circumferential direction of the first sleeve 23 with the locking member 22 being restricted to be axially fixed relative to the first sleeve 23. Therefore, the position of the locking member 22 is changeable, which facilitates quick recognition of the movable member 21 in the locked state or the released state, and thus increase the speed of releasing the stent 200.

Further, when the locking member 22 slides out of the second sliding groove 212, the movable member 21 is disengaged from the first sleeve 23 and the locking member 22, and the limiting member 12 moves away from the fixing member 11 when being driven by the movable member 21, and then releases the stent 200 to achieve the treatment of the vascular disease.

In a possible embodiment, with reference to FIG. 6, the movable member 21 includes a connecting portion 215 and an operating portion 216 connected to each other. The connecting portion 215 is provided with the first sliding groove 211 and the second sliding groove 212. The first sleeve 23 is mounted around an outer peripheral surface of the connecting portion 215. The connecting portion 215 is connected to the limiting member 12. The operating portion 216 is arranged apart from the first sleeve 23, and is configured to move under an external force, and drive the connecting portion 215 and the limiting member 12 to move away from or close to the fixing member 11.

Particularly, with reference to FIG. 6, the operating portion 216 may be a handheld structure. When the movable member 21 is released from the fastener 22, an operator holds the operating portion 216 with his or her hand and pulls the operating portion 216 towards a distal end of the stent delivery device 100. The operating portion 216 drives the connecting portion 215 to approach to the distal end of the stent delivery device 100, and thus the limiting member 12 approaches to the distal end of the stent delivery device 100 under the action of the connecting portion 215, so that the limiting member 12 gradually moves away from the fixing member 11, and thus the stent 200 is quickly released at the lesion site of the vessel.

In a possible embodiment, with reference to FIG. 6, the locking member 22 includes a toggle portion 221 and a sliding portion 222 connected to each other. The toggle portion 221 abuts against an outer peripheral surface of the first sleeve 23. The sliding portion 222 passes through the limiting hole 231 and abuts against an inner peripheral surface of the first sleeve 23. The toggle portion 221 is configured to slide relative to the first sleeve 23 under an external force, and drive the sliding portion 222 to slide in the first sliding groove 211 and the second sliding groove 212 through the limiting hole 231 of the first sleeve 23.

The sliding portion 222 may slide along the limiting hole 231 by sliding the toggle portion 221 by the operator, that is, the operator may make the locking member 22 lock or release the movable member 21 by operating the toggle portion 221. The operation is simple and convenient, with high feasibility, and quick implementation, and the efficiency of releasing the stent 200 is improved.

Further, the toggle portion 221 is detachably connected to the sliding portion 222, so that the locking member 22 may be conveniently and quickly mounted on the first sleeve 23.

In a possible embodiment, with reference to FIG. 6, the toggle portion 221 is snap-fit with the sliding portion 222. The toggle portion 221 includes a cover plate 223 and a hook 224 arranged on the cover plate 223. The cover plate 223 abuts against the outer surface of the first sleeve 23. The sliding portion 222 is provided with a first sliding portion 225 and a second sliding portion 226 connected to each other, with a through hole 227 extending through the first sliding portion 225 and the second sliding portion 226. The first sliding portion 225 is slidably engaged in the limiting hole 231 of the first sleeve 23, and the second sliding portion 226 is slidably engaged in the first sliding groove 211 and the second sliding groove 212 of the movable member 21. Particularly, the first sliding portion 225 is configured to slide along the limiting hole 231 of the first sleeve 23 when being driven by the toggle portion 221. In one embodiment, the first sliding portion 225 is configured to be at least partially accommodated in the limiting hole 231 of the first sleeve 23. The second sliding portion 226 is configured to slide in the first sliding groove 211 and the second sliding groove 211 of the movable member 21. The hook 224 is engaged with the second sliding portion 226 through the through hole 227, so that one end of the first sliding portion 225 abuts against the cover plate 223.

In other embodiments, the toggle portion 221 may be threadedly or magnetically connected to the sliding portion 222.

Further, with reference to FIG. 6, textures 228 is provided on an outer surface of the cover plate 223, such that when the operator manually toggles the cover plate 223, the textures 228 serves to avoid skidding.

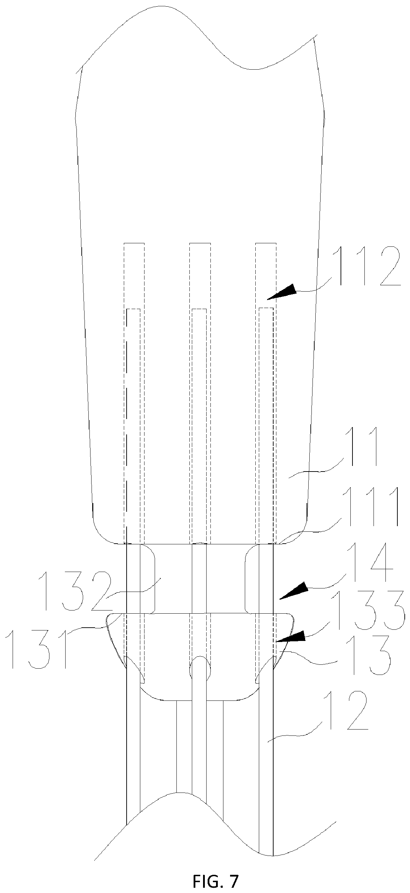

In combination with any one of the foregoing embodiments, in a possible embodiment, with reference to FIG. 7, the fixing assembly 1 further includes a guide member 13. The guide member 13 is closer to an operating end of the stent delivery device 100 relative to the fixing member 11. An accommodating space 14 is defined between the guide member 13 and the fixing member 11. The accommodating space 14 is configured to accommodate an end portion of the stent 200. One end of the limiting member 12 extends through the guide member 13 and extends into the accommodating space 14 so as to pass through the end portion of the stent 200 and abut against the fixing member 11.

Particularly, with reference to FIG. 7, the guide member 13 may be fixedly connected to the fixing member 11. The fixing member 11 is provided with a first surface 111 directly facing the guide member 13. The guide member 13 is provided with a second surface 131 directly facing the fixing member 11. A protrusion 132 may be provided on the second surface 131. The protrusion 132 may be connected to the first surface 111. A radial size of the protrusion 132 may be smaller than that of the first surface 111 and the second surface 131, that is, the protrusion 132 is sunken in the radial direction relative to the fixing member 11 and the guide member 13. The first surface 111, the second surface 131 and the outer peripheral surface of the protrusion 132 cooperatively define the accommodating space 14.

Further, the guide member 13 may be integrally formed with the fixing member 11 as one piece, that is, one end of the protrusion 132 is integrally formed with the guide member 13 and the other end of the protrusion 132 is integrally formed with the fixing member 11.

The fixing member 11 and the guide member 13 are provided in the fixing assembly 1, and the fixing member 11 works cooperatively with the limiting member 12 such that one end of the stent 200 is locked by the limiting member 12. The guide member 13 provides guiding for the limiting member 12, thus the limiting member 12 can move away from or close to the fixing member 11 in the first direction X. When the limiting member 12 moves away from the fixing member 11, one end of the stent 200 is disengaged from the limiting member 12 due to the blockage of the guide member 13, so that the stent 200 is released.

Further, with reference to FIG. 7, the first surface 111 is provided with a first hole 112. Particularly, the first hole 112 may be a blind hole. The first hole 112 extends along the first direction X. When the locking member 22 is in the first position or the second position, one end of the limiting member 12 extends into or exits from the first hole 112 when being driven by the movable member 21. By providing the first hole 112 in the fixing member 11, the limiting member 12 is capable of extending into the first hole 112 to achieve an engagement between the fixing member 11 and the limiting member 12, which facilitates the locking of the stent 2 by the limiting member 12 to prevent the stent 200 from being easily released.

Further, with reference to FIG. 7, the guide member 13 is provided with a second hole 133. The second hole 133 may extend through the guide member 13 along the first direction X. The second hole 133 is aligned with the first hole 112. The limiting member 12 extends through the second hole 133 and is slidably connected to the guide member 13.

Particularly, a proximal end of the limiting member 12 extends into the first hole 112 through the second hole 133. When the movable member 21 drives the limiting member 12 to move, the limiting member 12 slides along the second hole 133 to extend into or move out of the first hole 112, thereby locking or releasing the stent 200.

With the second hole 133 provided in the guide member 13, which serves to guide the limiting member 12, and the second hole 133 being aligned with the first hole 112, the limiting member 12 can accurately extend into the first hole 112.

Particularly, when the limiting member 12 moves away from the fixing member 11 when being driven by the movable member 21, an end portion of the limiting member 12 may be retracted into the second hole 133, and the stent 200 is released from the limiting member 12 due to the blockage of the guide member 13. The guide member 13 serves to facilitate the release of the stent 200 from the limiting member 12.

In a possible embodiment, with reference to FIG. 7, A plurality of the first holes 112 are provided. The plurality of first holes 112 are arranged along a circle. The second holes 133 has a number and an arrangement manner the same as those of the first holes 112. Each first hole 112 is corresponding to one second hole 133. A plurality of the limiting members 12 are provided, and the plurality of limiting members 12 is arranged in a tubular shape. Each of the limiting members 12 extends through one of the second holes 133 and is aligned with or inserted into one of the first holes 112.

Particularly, with reference to FIG. 3 and FIG. 7, the stent 200 is tubular. The proximal end of the stent 200 is provided with a plurality of hollowing portions, and the plurality of limiting members 12 respectively passes through the plurality of hollowing portions and is connected to the fixing member 11, so that the proximal end of the stent 200 is tightened and fixed on the limiting member 12.

The plurality of limiting members 12 are provided to fix the stent 200 along the circumferential direction, so that the proximal end of the stent 200 is tightened and the stent 200 is maintained in a tube shape. The firmness of the stent 200 fixed on the limiting member 12 may be improved.

In combination with any of the foregoing embodiments, in a possible embodiment, with reference to FIG. 3, the stent delivery device 100 further includes an inner core 3. The fixing member 11 and the movable member 21 are mounted around an outer periphery of the inner core 3. The fixing member 11 is fixed relative to the inner core 3. The movable member 21 is slidably connected to the inner core 3.

Particularly, the inner core 3 extends along the first direction X. The fixing member 11, the guide member 13, the locking member 22 and the movable member 21 are mounted around the outer periphery of the inner core 3 in sequence.

It should be understood that the inner core 3 may be of a hollow tubular shape, which can conduct a liquid into the vessel.

By providing the inner core 3, the inner core 3 is able to connect the fixing member 11, the guide member part 13, the locking member 22 with the movable member 21 together. Moreover, the inner core 3 also serves to guide the sliding of the movable member 21.

Further, with reference to FIG. 3, the stent delivery device 100 further includes an outer sheath 4. The outer sheath 4 is mounted around the outer periphery of the inner core 3. An accommodating cavity 40 is defined between the outer sheath 4 and the inner core 3. The stent 200 is compressed within the accommodating cavity 40.

The outer sheath 4 and the inner core 3 are provided to define the accommodating cavity 40. Since the accommodating cavity 40 is in an annular shape, the tubular stent 200 may be tightened within the accommodating cavity 40 in a tubular shape. On the one hand, a space within the stent delivery device 100 is fully utilized. On the other hand, the stent 200 is delivered after being tightened, which can reduce the profile of the stent delivery device 100, as a result, the interference of the stent delivery device 100 to the vessel is reduced. Moreover, the stent 200 is delivered in a tubular shape. The stent 200 is capable of easily returning to its original shape after being released, to achieve the treatment at the lesion site of the vessel.

In a possible embodiment, the inner core 3 and the outer sheath 4 are made of flexible materials, which may improve the flexibility of the stent delivery device 100, so that the stent delivery device 100 is able to deliver the stent 200 to a curved vessel, applications of the stent delivery device 100 are increased.

Particularly, the limiting member 12 is arranged within the accommodating cavity 40. The stent 200 is arranged between the limiting member 12 and the outer sheath 4.

In case that a plurality of limiting members 12 are provided, the plurality of limiting members 12 are annularly arranged within the accommodating cavity 40. The distal end of the stent 200 is mounted around the outer peripheries of limiting members 12.

As the limiting member 12 is accommodated between the inner core 3 and the outer sheath 4, and the limiting member 12 is arranged within the stent 200, the limiting member 12 would not affect the release of the distal end of the stent 200.

Further, the limiting member 12 is made of a flexible material, which further increases the flexibility of the stent delivery device 100, so that the stent delivery device 100 can deliver the stent 200 into the curved vessel, and thus the applications of the stent delivery device 100 are increased.

Particularly, the limiting elements 12 are separately arranged within the accommodating cavity 40. When the stent delivery device 100 enters a vessel which extends curvedly, the limiting elements 12 is able to be bent with the inner core 3 and the outer sheath 4, such that the stent delivery device 100 can deliver the stent 200 to the curved vessel.

Further, with reference to FIG. 3, one end of the outer sheath 4 abuts against the fixing member 11. The outer sheath 4 can slide relative to the inner core 3. When the outer sheath 4 moves away from the fixing member 11, a portion of the stent 200 which exposes from the outer sheath 4 expands under the action of its own tension.

Particularly, one end of the outer sheath 4 abuts against the fixing member 11 to enclose the limiting member 12, the stent 200, and the guide member 13 within the outer sheath 4, so as to prevent the stent 200 from being affected by the outside.

When the outer sheath 4 moves away from the fixing member 11, a portion of the proximal end of the stent 200 which exposes from the outer sheath 4 expands as the restraint of the outer sheath 4 is removed, while the stent 200 cannot be completely released since the limiting member 12 restricts the proximal end of the stent 200.

Further, with reference to FIG. 4, there is a retraction space 41 between the other end of the outer sheath 4 (a distal end of the outer sheath 4) and the releasing assembly 2. When the outer sheath 4 moves away from the fixing member 11, the other end of the outer sheath 4 (the distal end of the outer sheath 4) gradually extends into the retraction space 41. In other words, the retraction space 41 forms an avoiding space. When the outer sheath 4 moves away from the fixing member 11, the retraction space 41 prevents the other end of the outer sheath 4 (the distal end of the outer sheath 4) from abutting against the releasing assembly 2 which would result in the failure of retraction of the outer sheath 4.

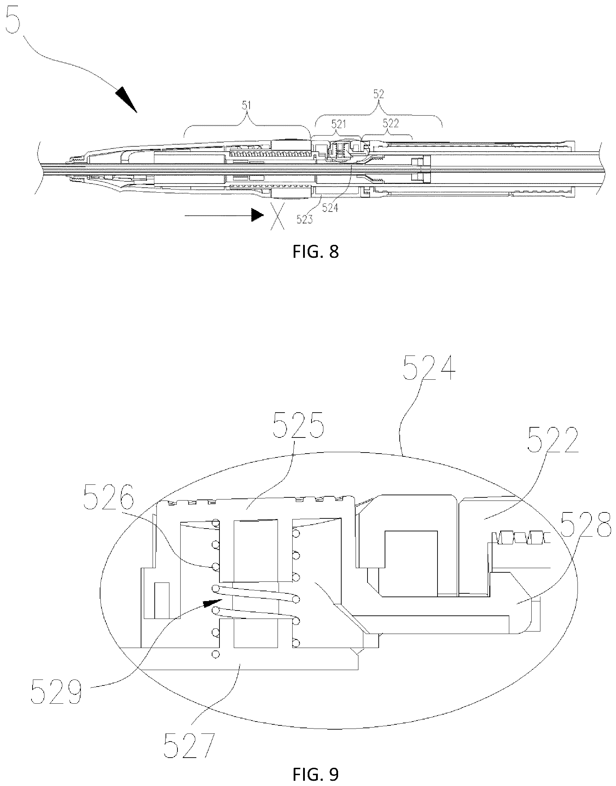

In a possible embodiment, with reference to FIG. 8, the stent delivery device 100 further includes an outer sheath sliding assembly 5, which is arranged between the fixing assembly 1 and the releasing assembly 2. The outer sheath sliding assembly 5 is mounted around the outer periphery of the outer sheath 4. The outer sheath sliding assembly 5 is configured to drive the outer sheath 4 to move away from the fixing member 11.

It should be understood that when the stent 200 is arranged within the vessel of a subject to be treated, the outer sheath sliding assembly 5 and the releasing assembly 2 are arranged outside the subject to be treated, so that the operator may control the expansion and release of the stent 200.

The outer sheath sliding assembly 5 is provided to control the outer sheath 4 to move away from the fixing member 11, which is convenient for operation and excellent in controllability.

In a possible embodiment, with reference to FIG. 8, the outer sheath sliding assembly 5 includes a second sleeve 51 and a driving assembly 52 threadedly connected to the second sleeve 51. The second sleeve 51 is mounted around the outer periphery of the outer sheath 4 and slidably connected to the outer sheath 4. The driving assembly 52 is mounted around the outer periphery of the outer sheath 4 and fixedly connected to the outer sheath 4. When the driving assembly 52 rotates relative to the second sleeve 51, the driving assembly 52 gradually moves away from the second sleeve 51, and the outer sheath 4 gradually moves away from the fixing member 11 under the action of the driving assembly 52, such that the proximal end of the stent 200 is gradually exposed from the outer sheath 4.

In a possible embodiment, with reference to FIG. 8, the driving assembly 52 includes a locking element 521 and a sliding sleeve 522. One end of the locking element 521 is threadedly connected to the second sleeve 51. The other end of the locking element 521 is detachably connected to the sliding sleeve 522. Particularly, the other end of the locking element 521 is snap-fit with the sliding sleeve 522. The locking element 521 is slidably connected to the outer sheath 4. The sliding sleeve 522 is fixedly connected to the outer sheath 4. When the locking element 521 is disengaged from the sliding sleeve 522, the sliding sleeve 522 can drive the outer sheath 4 to gradually move away from the fixing member 11.

By providing the locking element 521 and the sliding sleeve 522, with the locking element 521 being threadedly connected to the second sleeve 51, the locking element 521 is controlled to rotate relative to the second sleeve 51, so that the outer sheath 4 slowly retracts for a short distance, and the proximal end of the stent 200 gradually expands, while the locking element 521 is engaged with the sliding sleeve 522. When the locking element 521 is disengaged from the sliding sleeve 522, the sliding sleeve 522 is capable of driving the outer sheath 4 to retract rapidly for a longer distance after the restraint of the locking element 521 is removed, to allow the proximal end and the distal end of the stent 200 to expand.

Further, with reference to FIG. 8 to FIG. 9, the locking element 521 includes a third sleeve 523 and a switch 524 arranged on the third sleeve 523. The third sleeve 523 is mounted around the outer sheath 4 and slidable relative to the outer sheath 4. The switch 524 is connected to the third sleeve 523 and slidable relative thereto in a radial direction of the third sleeve 523. A buckling portion 528 of the switch 524 is snap-fit with the sliding sleeve 522. When the switch 524 slides to a pressed position, the buckling portion 528 of the switch 524 is disengaged from the sliding sleeve 522.

Further, the switch 524 is provided with a pressing portion 525, an elastic member 526 and a base 527 which are arranged along the radial direction of the third sleeve 523 in sequence. The pressing portion 525 is arranged on an outer peripheral surface of the third sleeve 523. The elastic member 526 elastically abuts between the pressing portion 525 and the base 521, so that a gap 529 is defined between the pressing portion 525 and the base 527. The buckling portion 528 extends from one end of the pressing portion 525 towards the sliding sleeve 522. When the pressing portion 525 abuts against the base 527, the switch 524 is in the pressed position. When the sliding sleeve 522 abuts against the third sleeve 523 and the switch 524 rebounds from the pressed position under the action of the elastic member 526, the buckling portion 528 of the switch 524 is engaged with the sliding sleeve 522 under the action of the pressing portion 525.

The stent delivery device 100 according to the present application can be operated with the following process.

During the operation, firstly, a vessel of a subject to be treated is punctured, a guide wire is inserted, and then the stent delivery device 100 is delivered into the aorta over the guide wire. Under X-ray fluoroscopy monitoring, the stent delivery device 100 is advanced to the vicinity of a lesion site of the vessel, the second sleeve 51 is fixed, the locking element 521 is rotated, and the outer sheath 4 is slowly retract, so that the proximal end of the stent 200 slowly expands, but the stent 200 does not fully expand yet since the proximal end of the stent 200 is still restrained by the limiting member 12. The stent 200 is moved to the most appropriate releasing position by moving the stent delivery device 100. The switch 524 on the locking element 521 is pressed, and in the meanwhile the sliding sleeve 522 is pulled backwards. A main body of the stent 200 expands completely. The locking member 22 is toggled to the second sliding groove 212, and the movable member 21 is pulled backwards to make the limiting member 12 move away from the fixing member 11 and further release the proximal end of the stent 200. The stent 200 is completely released from the stent delivery device 100, and the releasing process is completed. At the end, the stent delivery device 100 is withdrawn from the subject to be treated along the guide wire.

It should be understood that "pull backwards" and "retract" mentioned in the present application refer to a movement towards the operating end of the stent delivery device 100.

## Claims

1. A stent delivery device (100) for releasing a stent (200) into a vessel, comprising:
a fixing assembly (1), comprising a fixing member (11) and a limiting member (12), wherein one end of the limiting member (12) passes through the stent (200) and abuts against the fixing member (11) to lock the stent (200); and
a releasing assembly (2), comprising a movable member (21) and a locking member (22) connected to the movable member (21), wherein the movable member (21) is connected to an other end of the limiting member (12), **characterized in that**
an outer peripheral surface of the movable member (21) is provided with a first sliding groove (211) and a second sliding groove (212) communicating end to end, the first sliding groove (211) extends along a circumferential direction, the second sliding groove (212) extends along an axial direction (X), and one end of the locking member (22) is capable of sliding in the first sliding groove (211) and the second sliding groove (212);
the locking member (22) is slidably connected to the movable member (21) so that the locking member (22) is slidable relative to the movable member (21) along the first sliding groove (211) in the circumferential direction between a first position and a second position, and
when the locking member (22) is in the first position, the movable member (21) is fixed relative to the fixing member (11) along the axial direction (X) under the restriction of the locking member (22), and
when the locking member (22) slides to the second position, the movable member (21) is capable of driving the limiting member (12) to move along with it away from the fixing member (11) along the axial direction so as to release the stent (200).

2. The stent delivery device (100) according to claim 1, wherein the stent delivery device (100) has additional feature a) or features a) and b):
a) the releasing assembly (2) is provided with a first sleeve (23), an outer surface of the first sleeve (23) is provided with a first marker (201), and the locking member (22) is slidably connected to the first sleeve (23); when the locking member (22) is located at the first position, the locking member (22) is aligned with the first marker (201), and the limiting member (12) locks the stent (200); and when the locking member (22) is located at the second position, the locking member (22) is offset from the first marker (201), and the limiting member (12) releases the stent (200);
b) the outer surface of the first sleeve (23) is further provided with a second marker (202); and when the locking member (22) is located at the second position, the locking member (22) is aligned with the second marker (202).

3. The stent delivery device (100) according to claim 1 or 2, wherein the movable member (21) has a central axis (L), and the fixing member (11), the limiting member (12) and the movable member (21) are arranged along the direction (X) of the central axis (L).

4. The stent delivery device (100) according to claim 3, wherein the stent delivery device (100) has additional features d) or e):
d) the first position and the second position are located at the first sliding groove (211) and the second sliding groove (212), respectively;
e) the movable member (21) is provided with a first end surface (213), and the second sliding groove (212) extends through the first end surface (213) along the axial direction (X); and wherein when the movable member (21) moves away from the fixing member (11), the locking member (22) is capable of sliding out of the second sliding groove (212) until the locking member (22) is disengaged from the movable member (21).

5. The stent delivery device (100) according to claim 4, wherein when the stent delivery device (100) has the feature e), the releasing assembly (2) further comprises a first sleeve (23), the first sleeve (23) is mounted around an outer periphery of the movable member (21), the first sleeve (23) is provided with a limiting hole (231), the locking member (22) is arranged on the first sleeve (23), and the locking member (22) is slidably connected to the movable member (21) by extending of the one end of the locking member (22) through the limiting hole (231).

6. The stent delivery device (100) according to claim 5, wherein the stent delivery device (100) has additional feature f), g) or h):
f) the limiting hole (231) extends along the circumferential direction, the locking member (22) is capable of sliding from one end of the first sliding groove (211) to an other end of the first sliding groove (211) along the limiting hole (231), and an inner wall of the limiting hole (231) restricts the locking member (22) to be fixed relative to the first sleeve (23) in the axial direction (X);
g) the movable member (21) comprises a connecting portion (215) and an operating portion (216) connected to each other, the connecting portion (215) is provided with the first sliding groove (211) and the second sliding groove (212), the first sleeve (23) is mounted around an outer peripheral surface of the connecting portion (215), the connecting portion (215) is connected to the limiting member (12), the operating portion (216) is arranged outside the first sleeve (23), and the operating portion (216) is configured to move under an external force, and drive the connecting portion (215) and the limiting member (12) to move away from or close to the fixing member (11);
h) the locking member (22) comprises a toggle portion (221) and a sliding portion (222) connected to each other, the toggle portion (221) abuts against an outer peripheral surface of the first sleeve (23), the sliding portion (222) passes through the limiting hole (231) and abuts against an inner peripheral surface of the first sleeve (23), and the toggle portion (221) is configured to slide relative to the first sleeve (23) under an external force, so as to drive the sliding portion (222) to slide along the limiting hole (231) of the first sleeve (23).

7. The stent delivery device (100) according to claim 6, wherein when the stent delivery device (100) has the feature h), the stent delivery device (100) has further additional feature i), or j):
i) the toggle portion (221) and the sliding portion (222) are detachably connected;
j) the toggle portion (221) comprises a cover plate (223) and a hook (224) arranged on the cover plate (223), the cover plate (223) abuts against an outer surface of the first sleeve (23), the sliding portion (222) is provided with a first sliding portion (225) and a second sliding portion (226) connected to each other, with a through hole (227) extending through the first sliding portion (225) and the second sliding portion (226), the first sliding portion (225) is slidably engaged in the limiting hole (231) of the first sleeve (23), and the second sliding portion (226) is slidably engaged in the first sliding groove (211) and the second sliding groove (212) of the movable member (21); and the hook (224) is engaged with the second sliding portion (226) through the through hole (227), so that one end of the first sliding portion (225) abuts against the cover plate (223).

8. The stent delivery device (100) according to any one of claims 1 to 2 or 4 to 7, wherein the fixing assembly (1) further comprises a guide member (13), the guide member (13) is arranged opposite to the fixing member (11) with an accommodating space (14) defined between the guide member (13) and the fixing member (11), and the accommodating space (14) is configured to accommodating an end portion of the stent (200), and wherein one end of the limiting member (12) passes through the guide member (13) and extends into the accommodating space (14), and passes through the end portion of the stent (200) and is connected to the fixing member (11).

9. The stent delivery device (100) according to claim 8, wherein the stent delivery device (100) has additional feature k), or features k) and l), or features k, l) and m):
k) the fixing member (11) is provided with a first surface (111) opposite to the guide member (13), the first surface (111) is provided with at least one first hole (112); and when the locking member (22) is in the first position or the second position, one end of the limiting member (12) extends into or exits from the first hole (112) when being driven by the movable member (21);
l) the guide member (13) is provided with at least one second hole (133), and the second hole (133) is aligned with the first hole (112), and the limiting member (12) passes through the second hole (133) and is slidably connected to the guide member (13);
m) when the limiting member (12) moves away from the fixing member (11) when being driven by the movable member (21), an end portion of the limiting member (12) is capable of retracting into the second hole (133), and the end portion of the stent (200) is disengaged from the limiting member (12) due to blockage of the guide member (13).

10. The stent delivery device (100) according to any one of claims 1 to 2, 4 to 7, or 9, wherein the stent delivery device (100) further comprises additional feature n) or features n) and o):
n) an inner core (3), wherein the fixing member (11) and the movable member (21) are mounted around an outer periphery of the inner core (3), the fixing member (11) is fixed relative to the inner core (3), and the movable member (21) is slidably connected to the inner core (3);
o) an outer sheath (4), wherein the outer sheath (4) is mounted around the outer periphery of the inner core (3), an accommodating cavity (40) is defined between the outer sheath (4) and the inner core (3), and the stent (200) is compressed within the accommodating cavity (40).

11. The stent delivery device (100) according to claim 10, wherein when the stent delivery device (100) has the features n) and o), the stent delivery device (100) has further additional feature p), q) or r):
p) the inner core (3) and the outer sheath (4) are made of flexible material;
q) the limiting member (12) is arranged within the accommodating cavity (40), and the stent (200) is arranged between the limiting member (12) and the outer sheath (4);
r) one end of the outer sheath (4) abuts against the fixing member (11), the outer sheath (4) is capable of sliding relative to the inner core (3); and wherein when the outer sheath (4) moves away from the fixing member (11), a portion of the stent (200) which exposes from the outer sheath (4) expands under an action of its own tension.

12. The stent delivery device (100) according to claim 11, wherein when the stent delivery device (100) has the feature r), a retraction space (41) is provided between an other end of the outer sheath (4) and the releasing assembly (2); and wherein when the outer sheath (4) moves away from the fixing member (11), the other end of the outer sheath (4) extends into the retraction space (41).

13. The stent delivery device (100) according to claim 12, wherein the stent delivery device (100) further comprises additional feature s) or features s) and t):
s) an outer sheath sliding assembly (5), wherein the outer sheath sliding assembly (5) is arranged between the fixing assembly (1) and the releasing assembly (2), the outer sheath sliding assembly (5) is mounted around an outer periphery of the outer sheath (4), and the outer sheath sliding assembly (5) is configured to drive the outer sheath (4) to move away from or close to the fixing member (11);
t) the outer sheath sliding assembly (5) comprises a second sleeve (51) and a driving assembly (52) threadedly connected to the second sleeve (51), and the second sleeve (51) is mounted around the outer periphery of the outer sheath (4) and slidably connected to the outer sheath (4); the driving assembly (52) is mounted around the outer periphery of the outer sheath (4) and fixedly connected to the outer sheath (4); and wherein when the driving assembly (52) rotates relative to the second sleeve (51), the driving assembly (52) gradually moves away from the second sleeve (51), and the outer sheath (4) driven by the driving assembly (52) gradually moves away from the fixing member (11).

14. The stent delivery device (100) according to claim 13, wherein when the stent delivery device (100) has the features s) and t), the driving assembly (52) comprises a locking element (521) and a sliding sleeve (522), one end of the locking element (521) is threadedly connected to the second sleeve (51), the locking element (521) is slidably connected to the outer sheath (4), and an other end of the locking element (521) is detachably connected to the sliding sleeve (522); and the sliding sleeve (522) is fixedly connected to the outer sheath (4), and wherein when the locking member (22) is disengaged from the sliding sleeve (522), the sliding sleeve (522) is capable of driving the outer sheath (4) to gradually move away from the fixing member (11).

15. The stent delivery device (100) according to claim 14, wherein the stent delivery device (100) has additional feature u) or features u) and v);
u) the locking member (22) comprises a third sleeve (523) and a switch (524) provided on the third sleeve (523), the third sleeve (523) is mounted around the outer sheath (4) and slidable relative to the outer sheath (4), the switch (524) is slidably connected to the third sleeve (523) along a radial direction of the third sleeve (523), and the switch (524) has a buckling portion (528) engaged with the sliding sleeve (522); and wherein when the switch (524) slides to a pressed position, the buckling portion (528) of the switch (524) is disengaged from the sliding sleeve (522);
v) the switch (524) is provided with a pressing portion (525), an elastic member (526) and a base (527) arranged in sequence along a radial direction of the third sleeve (523), the pressing portion (525) is arranged on an outer peripheral surface of the third sleeve (523), the elastic member (526) elastically abuts between the pressing portion (525) and the base (527) with a gap (529) defined between the pressing portion (525) and the base (527); the buckling portion (528) extends towards the sliding sleeve (522) from one end of the pressing portion (525); and wherein when the pressing portion (525) abuts against the base (527), the switch (524) is in the pressed position; while when the sliding sleeve (522) abuts against the third sleeve (523) and the switch (524) rebounds from the pressed position under an action of the elastic member (526), the buckling portion (528) of the switch (524) is engaged with the sliding sleeve (522) under an action of the pressing portion (525).

## Patentansprüche

1. Eine Stentzufuhrvorrichtung (100) zum Freigeben eines Stents (200) in ein Gefäß, umfassend:
eine Befestigungsanordnung (1), umfassend ein Befestigungselement (11) und ein Begrenzungselements (12), wobei ein Ende des Begrenzungselements (12) durch den Stent (200) tritt und an dem Befestigungselement (11) anstößt, um den Stent (200) zu verriegeln; und
eine Freigabeanordnung (2), umfassend ein bewegbares Element (21) und ein Verriegelungselement (22), das mit dem bewegbaren Element (21) verbunden ist, wobei das bewegbare Element (21) mit einem anderen Ende des Begrenzungselements (12) verbunden ist, **dadurch gekennzeichnet, dass** eine äußere Umfangsoberfläche des bewegbaren Elements (21) mit einer ersten Gleitnut (211) und
einer zweiten Gleitnut (212) versehen ist, die an ihren Enden miteinander in Verbindung stehen, die erste Gleitnut (211) sich entlang einer umlaufenden Richtung erstreckt, die zweite Gleitnut (212) sich entlang einer axialen Richtung (X) erstreckt und ein Ende des Verriegelungselements (22) in der Lage ist, in der ersten Gleitnut (211) und der zweiten Gleitnut (212) zu gleiten;
das Verriegelungselement (22) gleitend mit dem bewegbaren Element (21) verbunden ist, sodass das Verriegelungselement (22) gleitbar relativ zu dem bewegbaren Element (21) entlang der ersten Gleitnut (211) in der umlaufenden Richtung zwischen einer ersten Position und einer zweiten Position gleitbar ist, und
wenn sich das Verriegelungselement (22) in der ersten Position befindet, das bewegbare Element (21) bezogen auf das Befestigungselement (11) entlang der axialen Richtung (X) mit der Einschränkung durch das Verriegelungselement (22) befestigt ist, und
wenn das Verriegelungselement (22) in die zweite Position gleitet, ist das bewegbare Element (21) in der Lage, das Begrenzungselement (12) so anzutreiben, dass es sich mit ihm weg von dem Befestigungselement (11) entlang der axialen Richtung bewegt, um den Stent (200) freizugeben.

2. Die Stentzufuhrvorrichtung (100) gemäß Anspruch 1, wobei die Stentzufuhrvorrichtung (100) ein zusätzliches Merkmal a) oder Merkmale a) und b) aufweist:
a) die Freigabeanordnung (2) ist mit einer ersten Hülse (23) versehen, eine äußere Oberfläche der ersten Hülse (23) ist mit einer ersten Markierung (201) versehen und das Verriegelungselement (22) ist gleitbar mit der ersten Hülse (23) verbunden; wenn sich das Verriegelungselement (22) in der ersten Position befindet, ist das Verriegelungselement (22) mit der ersten Markierung (201) ausgerichtet, und das Begrenzungselement (12) verriegelt den Stent (200); und wenn sich das Verriegelungselement (22) in der zweiten Position befindet, ist das Verriegelungselement (22) versetzt zu der ersten Markierung (201), und das Begrenzungselement (12) gibt den Stent (200) frei;
b) die äußere Oberfläche der ersten Hülse (23) ist des Weiteren mit einer zweiten Markierung (202) versehen; und wenn sich das Verriegelungselement (22) in der zweiten Position befindet, ist das Verriegelungselement (22) mit der zweiten Markierung (202) ausgerichtet.

3. Die Stentzuführvorrichtung (100) gemäß Anspruch 1 oder 2, wobei das bewegbare Element (21) eine Mittelachse (L) aufweist, und das Befestigungselement (11), das Begrenzungselement (12) und das bewegbare Element (21) sind entlang der Richtung (X) der Mittelachse (L) angeordnet.

4. Die Stentzuführvorrichtung (100) gemäß Anspruch 3, wobei die Stentzuführvorrichtung (100) zusätzliche Merkmale d) oder e) aufweist:
d) die erste Position und die zweite Position befinden sich an der ersten Gleitnut (211) bzw. der zweiten Gleitnut (212);
e) das bewegbare Element (21) ist mit einer ersten Endoberfläche (213) versehen und die zweite Gleitnut (212) erstreckt sich durch die erste Endoberfläche (213) entlang der axialen Richtung (X); und wobei dann, wenn sich das bewegbare Element (21) von dem Befestigungselement (11) wegbewegt, das Verriegelungselement (22) in der Lage ist, aus der zweiten Gleitnut (212) herauszugleiten, bis sich das Verriegelungselement (22) aus dem Eingriff mit dem bewegbaren Element (21) löst.

5. Die Stentzuführvorrichtung (100) gemäß Anspruch 4, wobei dann, wenn die Stentzuführvorrichtung (100) das Merkmal e) aufweist, die Freigabeanordnung (2) des Weiteren eine erste Hülse (23) umfasst, die erste Hülse (23) um einen äußeren Umfang des bewegbaren Elements (21) montiert ist, die erste Hülse (23) mit einem Begrenzungsloch (231) versehen ist, das Verriegelungselement (22) auf der ersten Hülse (23) angeordnet ist, und das Verriegelungselement (22) gleitbar mit dem bewegbaren Element (21) verbunden ist, indem sich das eine Ende des Verriegelungselements (22) durch das Begrenzungsloch (231) erstreckt.

6. Die Stentzuführvorrichtung (100) gemäß Anspruch 5, wobei die Stentzuführvorrichtung (100) ein zusätzliches Merkmal f), g) oder h) aufweist:
f) das Begrenzungsloch (231) erstreckt sich in der umlaufenden Richtung, das Verriegelungselement (22) ist in der Lage, von einem Ende der ersten Gleitnut (211) zu einem anderen Ende der ersten Gleitnut (211) entlang des Begrenzungslochs (231) zu gleiten, und eine Innenwand des Begrenzungslochs (231) schränkt das Verriegelungselement (22) ein, sodass es bezogen auf die erste Hülse (23) in der axialen Richtung (X) befestigt ist;
g) das bewegbare Element (21) umfasst einen Verbindungsbereich (215) und einen Betriebsbereich (216), die miteinander verbunden sind, der Verbindungsbereich (215) ist mit der ersten Gleitnut (211) und der zweiten Gleitnut (212) versehen, die erste Hülse (23) ist um eine äußere Umfangsoberfläche des Verbindungsbereichs (215) angeordnet, der Verbindungsbereich (215) ist mit dem Begrenzungselement (12) verbunden, der Betriebsbereich (216) ist außerhalb der ersten Hülse (23) angeordnet, und der Betriebsbereich (216) ist so konfiguriert, dass er sich unter einen externen Kraft bewegt und den Verbindungsbereich (215) und das Begrenzungselement (12) antreibt, dass sie sich weg von oder in die Nähe von dem Befestigungselement (11) bewegen;
h) das Verriegelungselement (22) umfasst einen Gelenkbereich (221) und einen Gleitbereich (222) die miteinander verbunden sind, der Gelenkbereich (211) stößt an eine äußere Umfangsoberfläche der ersten Hülse (23) an, der Gleitbereich (222) tritt durch das Begrenzungsloch (231) und stößt an eine innere Umfangsoberfläche der ersten Hülse (23) an, und der Gelenkbereich (221) ist so konfiguriert, dass er bezogen auf die erste Hülse (23) unter einer externen Kraft gleitet, um den Gleitbereich (222) so anzutreiben, dass er entlang des Begrenzungslochs (231) der ersten Hülse (23) gleitet.

7. Die Stentzuführvorrichtung (100) gemäß Anspruch 6, wobei wenn die Stentzuführvorrichtung (100) das Merkmal h) aufweist, die Stentzuführvorrichtung (100) ein weiteres zusätzliches Merkmal i) oder j) aufweist:
i) der Gelenkbereich (221) und der Gleitbereich (222) sind lösbar verbunden;
j) der Gelenkbereich (221) umfasst eine Abdeckplatte (223) und einen Haken (224), der auf der Abdeckplatte (223) angeordnet ist, die Abdeckplatte (223) stößt an einer äußeren Oberfläche der ersten Hülse (23) an, der Gleitbereich (222) ist mit einem ersten Gleitbereich (225) und einem zweiten Gleitbereich (226) versehen, die miteinander verbunden sind, mit einem Durchgangsloch (227) das sich durch den ersten Gleitbereich (225) und den zweiten Gleitbereich (226) erstreckt, der erste Gleitbereich (225) steht in gleitendem Eingriff mit dem Begrenzungsloch (231) der ersten Hülse (23), und der zweite Gleitbereich (226) steht in gleitendem Eingriff mit der ersten Gleitnut (211) und der zweiten Gleitnut (212) des bewegbaren Elements (21); und der Haken (224) steht in Eingriff mit dem zweiten Gleitbereich (226) durch das Durchgangsloch (227), sodass ein Ende des ersten Gleitbereichs (225) an der Abdeckplatte (223) anstößt.

8. Die Stentzuführvorrichtung (100) nach einem der Ansprüche 1 bis 2 oder 4 bis 7, wobei die Befestigungsanordnung (1) des Weiteren ein Führungselement (13) umfasst, das Führungselement (13) ist gegenüber des Befestigungselements (11) mit einem Aufnahmeabstand (14), der zwischen dem Führungselement (13) und dem Befestigungselement (11) definiert ist, angeordnet, und der Aufnahmeabstand (14) ist so konfiguriert, dass er einen Endbereich des Stents (200) aufnimmt, und wobei ein Ende des Begrenzungselements (12) durch das Führungselement (13) tritt und sich in den Aufnahmebereich (14) erstreckt und durch den Endbereich des Stents (200) tritt und mit dem Befestigungselement (11) verbunden ist.

9. Die Stentzuführvorrichtung (100) gemäß Anspruch 8, wobei die Stentzufuhrvorrichtung (100) das zusätzliche Merkmal k), oder Merkmale k) und l), oder Merkmale k), l) und m) aufweist:
k) das Befestigungselement (11) ist mit einer ersten Oberfläche (111) gegenüber des Führungselements (13) versehen, die erste Oberfläche (111) ist mit mindestens einem ersten Loch (112) versehen; und wenn sich das Verriegelungselement (22) in der ersten Position oder der zweiten Position befindet, erstreckt sich ein Ende des Begrenzungselements (12) in das erste Loch (112) oder tritt aus ihm heraus, wenn es durch das bewegbare Element (21) angetrieben wird;
l) das Führungselement (13) ist mit mindestens einem zweiten Loch (133) versehen, und das zweite Loch (133) ist mit dem ersten Loch (112) ausgerichtet, und das Begrenzungselement (12) tritt durch das zweite Loch (133) und ist gleitend mit dem Führungselement (13) verbunden;
m) wenn sich das Begrenzungselement (12) von dem Befestigungselement (11) wegbewegt, wenn es von dem bewegbaren Element (21) angetrieben wird, ist ein Endbereich des Begrenzungselements (12) in der Lage, in das zweite Loch (133) einzufahren, und der Endbereich des Stents (200) löst sich aufgrund der Blockade des Führungselements (13) aus dem Eingriff mit dem Begrenzungselement (12).

10. Die Stentzuführvorrichtung (100) gemäß einem der Ansprüche 1 bis 2, 4 bis 7 oder 9, wobei die Stentzuführvorrichtung (100) des Weiteren das zusätzliche Merkmal n) oder Merkmale n) und o) umfasst:
n) einen inneren Kern (3), wobei das Befestigungselement (11) und das bewegbare Element (21) um einen äußeren Umfang des inneren Kerns (3) angeordnet sind, das Befestigungselement (11) ist bezogen auf den inneren Kern (3) befestigt, und das bewegbare Element (21) ist gleitbar mit dem inneren Kern (3) verbunden;
o) eine äußere Ummantelung (4), wobei die äußere Ummantelung (4) um den äußeren Umfang des inneren Kerns (3) angeordnet ist, ein Aufnahmehohlraum (40) ist zwischen der äußeren Ummantelung (4) und dem inneren Kern (3) definiert, und der Stent (200) ist innerhalb des Aufnahmehohlraums (40) komprimiert.

11. Die Stentzuführvorrichtung (100) gemäß Anspruch 10, wobei dann, wenn die Stentzufuhrvorrichtung (100) die Merkmale n) und o) aufweist, die Stentzufuhrvorrichtung das weitere zusätzliche Merkmal p) q) oder r) aufweist:
p) der innere Kern (3) und die äußere Ummantelung (4) sind aus einem flexiblen Material hergestellt;
q) das Begrenzungselement (12) ist innerhalb des Aufnahmehohlraums (40) angeordnet, und der Stent (200) ist zwischen dem Begrenzungselement (12) und der äußeren Ummantelung (4) angeordnet;
r) ein Ende der äußeren Ummantelung (4) stößt an dem Befestigungselement (11) an, die äußere Ummantelung (4) ist in der Lage, bezogen auf den inneren Kern (3) zu gleiten; und wobei sich dann, wenn sich die äußere Ummantelung (14) von dem Befestigungselement (11) wegbewegt, ein Bereich des Stents (200), der von der äußeren Ummantelung (4) freigegeben ist, unter einer Handlung seiner eigenen Spannung ausdehnt.

12. Die Stentzuführvorrichtung (100) gemäß Anspruch 11, wobei dann, wenn die Stentzufuhrvorrichtung (100) das Merkmal r) aufweist, ein Rückziehbereich (41) zwischen einem anderen Ende der äußeren Ummantelung (4) und der Freigabeanordnung (2) vorgesehen ist; und wobei dann, wenn sich die äußere Ummantelung (4) von dem Befestigungselement (11) wegbewegt, das andere Ende der äußeren Ummantelung (4) in den Rückziehbereich (41) erstreckt.

13. Die Stentzuführvorrichtung (100) gemäß Anspruch 12, wobei die Stentzufuhrvorrichtung (100) des Weiteren das zusätzliche Merkmal s) oder Merkmale s) und t) umfasst:
s) eine äußere Ummantelungsgleitanordnung (5), wobei die äußere Ummantelungsgleitanordnung (5) zwischen der Befestigungsanordnung (1) und der Freigabeanordnung (2) angeordnet ist, die äußere Ummantelungsgleitanordnung (5) ist um einen äußeren Umfang der äußeren Ummantelung (4) angeordnet, und die äußere Ummantelungsgleitanordnung (5) ist so konfiguriert, dass sie die äußere Ummantelung (4) so antreibt, dass sie sich weg von oder in die Nähe von dem Befestigungselement (11) bewegt;
t) die äußere Ummantelungsgleitanordnung (5) umfasst eine zweite Hülse (51) und eine Antriebsanordnung (52), die über ein Gewinde mit der zweiten Hülse (51) verbunden ist, und die zweite Hülse (51) ist um den äußeren Umfang der äußeren Ummantelung (4) angeordnet und gleitbar mit der äußeren Ummantelung (4) verbunden; die Antriebsanordnung (52) ist um den Außenumfang der äußeren Ummantelung (4) angeordnet und fest mit der äußeren Ummantelung (4) verbunden; und wobei sich dann, wenn sich die Antriebsanordnung (52) bezogen auf die zweite Hülse (51) dreht, die Antriebsanordnung (52) allmählich von der zweiten Hülse (51) wegbewegt, und die äußere Ummantelung (4), die von der Antriebsanordnung (52) angetrieben wird, bewegt sich allmählich von dem Befestigungselement (11) weg.

14. Die Stentzuführvorrichtung (100) gemäß Anspruch 13, wobei dann, wenn die Stentzufuhrvorrichtung (100) die Merkmale s) und t) aufweist, die Antriebsanordnung (52) ein Verriegelungselement (521) und eine Gleithülse (522) umfasst, ein Ende des Verriegelungselements (521) über ein Gewinde mit der zweiten Hülse (51) verbunden ist, das Verriegelungselement (521) gleitend mit der äußeren Ummantelung (4) verbunden ist und ein anderes Ende des Verriegelungselements (521) lösbar mit der Gleithülse (522) verbunden ist; und die Gleithülse (522) ist fest mit der äußeren Ummantelung (4) verbunden, und wobei dann, wenn das Verriegelungselement (22) aus dem Eingriff mit der Gleithülse (522) genommen wird, die Gleithülse (522) in der Lage ist, die äußere Ummantelung (4) anzutreiben, sodass sie sich allmählich von dem Befestigungselement (11) wegbewegt.

15. Die Stentzuführvorrichtung (100) gemäß Anspruch 14, wobei die Stentzuführvorrichtung (100) ein zusätzliches Merkmal u) oder Merkmale u) und v) aufweist;
u) das Verriegelungselement (22) umfasst eine dritte Hülse (523) und einen Schalter (524), der an der dritten Hülse (523) vorgesehen ist, die dritte Hülse (523) ist um die äußere Ummantelung (4) herum angeordnet und ist gleitbar bezogen auf die äußere Ummantelung (4), der Schalter (524) ist gleitbar mit der dritten Hülse (523) entlang einer radialen Richtung der dritten Hülse (523) verbunden, und der Schalter (524) weist einen Gelenkbereich (528) auf, der mit der Gleithülse (522) in Eingriff steht; und wobei dann, wenn der Schalter (524) in eine Pressposition gleitet, sich der Gelenkbereich (528) des Schalters (524) aus dem Eingriff mit der Gleithülse (522) löst;
v) der Schalter (524) ist mit einem Pressbereich (525), einem elastischen Element (526) und einer Basis (527) versehen, die in Reihe entlang einer radialen Richtung der dritten Hülse (523) angeordnet sind, der Pressbereich (525) ist an einer äußeren Umfangsoberfläche der dritten Hülse (523) angeordnet, das elastische Element (526) stößt elastisch zwischen dem Pressbereich (525) und der Basis (527) mit einem Spalt (529), der zwischen dem Pressbereich (525) und der Basis (527) definiert ist, an; der Gelenkbereich (528) erstreckt sich in Richtung der Gleithülse (522) von einem Ende des Pressbereichs (525); und wobei sich dann, wenn der Pressbereich (525) an die Basis (527) angrenzt, der Schalter (524) in der Pressposition befindet, während dann, wenn die Gleithülse (522) an die dritte Hülse (523) anstößt und der Schalter (524) aus der Pressposition aufgrund einer Aktion des elastischen Elements (526) zurückspringt, der Gelenkbereich (528) des Schalters (524) aufgrund einer Aktion des Pressbereichs (525) mit der Gleithülse (522) in Eingriff gelangt.

## Revendications

1. Dispositif de transport de stent (100) permettant de libérer un stent (200) dans un vaisseau, comportant :
un ensemble de fixation (1), comportant un élément de fixation (11) et un élément de limitation (12), dans lequel une extrémité de l'élément de limitation (12) traverse le stent (200) et bute contre l'élément de fixation (11) pour verrouiller le stent (200) ; et
un ensemble de libération (2), comportant un élément mobile (21) et un élément de verrouillage (22) relié à l'élément mobile (21), dans lequel l'élément mobile (21) est relié à une autre extrémité de l'élément de limitation (12), **caractérisé en ce que**
une surface périphérique extérieure de l'élément mobile (21) est dotée d'une première rainure de coulissement (211) et d'une seconde rainure de coulissement (212) communiquant bout-à-bout, la première rainure de coulissement (211) s'étend le long d'une direction circonférentielle, la seconde rainure de coulissement (212) s'étend le long d'une direction axiale (X) et une extrémité de l'élément de verrouillage (22) est capable de coulisser dans la première rainure de coulissement (211) et la seconde rainure de coulissement (212) ;
l'élément de verrouillage (22) est relié de manière coulissante à l'élément mobile (21), de sorte que l'élément de verrouillage (22) puisse coulisser par rapport à l'élément mobile (21) le long de la première rainure de coulissement (211) dans la direction circonférentielle entre une première position et une seconde position et
lorsque l'élément de verrouillage (22) est dans la première position, l'élément mobile (21) est fixe par rapport à l'élément de fixation (11) le long de la direction axiale (X) sous la restriction de l'élément de verrouillage (22) et
lorsque l'élément de verrouillage (22) coulisse jusqu'à la seconde position, l'élément mobile (21) est capable d'amener l'élément de limitation (12) à s'éloigner avec lui de l'élément de fixation (11) le long de la direction axiale, de manière à libérer le stent (200).

2. Dispositif de transport de stent (100) selon la revendication 1, dans lequel le dispositif de transport de stent (100) a la caractéristique a) ou les caractéristiques a) et b) supplémentaires :
a) l'ensemble de libération (2) est doté d'un premier manchon (23), une surface extérieure du premier manchon (23) est dotée d'un premier repère (201) et l'élément de verrouillage (22) est relié de manière coulissante au premier manchon (23) ; lorsque l'élément de verrouillage (22) est situé à la première position, l'élément de verrouillage (22) est aligné sur le premier repère (201) et l'élément de limitation (12) verrouille le stent (200) ; et lorsque l'élément de verrouillage (22) est situé à la seconde position, l'élément de verrouillage (22) est décalé du premier repère (201) et l'élément de limitation (12) libère le stent (200) ;
b) la surface extérieure du premier manchon (23) est en outre dotée d'un second repère (202) ; et lorsque l'élément de verrouillage (22) est situé à la seconde position, l'élément de verrouillage (22) est aligné sur le second repère (202).

3. Dispositif de transport de stent (100) selon la revendication 1 ou 2, dans lequel l'élément mobile (21) a un axe central (L) et l'élément de fixation (11), l'élément de limitation (12) et l'élément mobile (21) sont agencés le long de la direction (X) de l'axe central (L).

4. Dispositif de transport de stent (100) selon la revendication 3, dans lequel le dispositif de transport de stent (100) a les caractéristiques d) ou e) supplémentaires :
d) la première position et la seconde position sont situées respectivement au niveau de la première rainure de coulissement (211) et de la seconde rainure de coulissement (212) ;
e) l'élément mobile (21) est doté d'une première surface d'extrémité (213) et la seconde rainure de coulissement (212) s'étend à travers la première surface d'extrémité (213) le long de la direction axiale (X) ; et dans lequel, lorsque l'élément mobile (21) s'éloigne de l'élément de fixation (11), l'élément de verrouillage (22) est capable de coulisser hors de la seconde rainure de coulissement (212) jusqu'à ce que l'élément de verrouillage (22) soit désolidarisé de l'élément mobile (21).

5. Dispositif de transport de stent (100) selon la revendication 4, dans lequel, lorsque le dispositif de transport de stent (100) a la caractéristique e), l'ensemble de libération (2) comporte en outre un premier manchon (23), le premier manchon (23) est monté autour d'une périphérie extérieure de l'élément mobile (21), le premier manchon (23) est doté d'un trou de limitation (231), l'élément de verrouillage (22) est agencé sur le premier manchon (23) et l'élément de verrouillage (22) est relié de manière coulissante à l'élément mobile (21) par extension de la première extrémité de l'élément de verrouillage (22) à travers le trou de limitation (231).

6. Dispositif de transport de stent (100) selon la revendication 5, dans lequel le dispositif de transport de stent (100) a la caractéristique f), g) ou h) supplémentaire :
f) le trou de limitation (231) s'étend le long de la direction circonférentielle, l'élément de verrouillage (22) est capable de coulisser d'une extrémité de la première rainure de coulissement (211) à une autre extrémité de la première rainure de coulissement (211) le long du trou de limitation (231) et une paroi intérieure du trou de limitation (231) limite l'élément de verrouillage (22) à être fixe par rapport au premier manchon (23) dans la direction axiale (X) ;
g) l'élément mobile (21) comporte une portion de liaison (215) et une portion de fonctionnement (216) reliées l'une à l'autre, la portion de liaison (215) est dotée de la première rainure de coulissement (211) et de la seconde rainure de coulissement (212), le premier manchon (23) est monté autour d'une surface périphérique extérieure de la portion de liaison (215), la portion de liaison (215) est reliée à l'élément de limitation (12), la portion de fonctionnement (216) est agencée à l'extérieur du premier manchon (23) et la portion de fonctionnement (216) est conçue pour se déplacer sous l'effet d'une force externe et amener la portion de liaison (215) et l'élément de limitation (12) à s'éloigner ou à se rapprocher de l'élément de fixation (11) ;
h) l'élément de verrouillage (22) comporte une portion de bascule (221) et une portion de coulissement (222) reliées l'une à l'autre, la portion de bascule (221) bute contre une surface périphérique extérieure du premier manchon (23), la portion de coulissement (222) traverse le trou de limitation (231) et bute contre une surface périphérique intérieure du premier manchon (23) et la portion de bascule (221) est conçue pour coulisser par rapport au premier manchon (23) sous l'effet d'une force externe, de manière à amener la portion de coulissement (222) à coulisser le long du trou de limitation (231) du premier manchon (23).

7. Dispositif de transport de stent (100) selon la revendication 6, dans lequel, lorsque le dispositif de transport de stent (100) a la caractéristique h), le dispositif de transport de stent (100) a en outre la caractéristique i) ou j) supplémentaire :
i) la portion de bascule (221) et la portion de coulissement (222) sont reliées de manière détachable ;
j) la portion de bascule (221) comporte une plaque de recouvrement (223) et un crochet (224) agencé sur la plaque de recouvrement (223), la plaque de recouvrement (223) bute contre une surface extérieure du premier manchon (23), la portion de coulissement (222) est dotée d'une première portion de coulissement (225) et d'une seconde portion de coulissement (226) reliées l'une à l'autre, un trou traversant (227) s'étendant à travers la première portion de coulissement (225) et la seconde portion de coulissement (226), la première portion de coulissement (225) est en prise coulissante dans le trou de limitation (231) du premier manchon (23) et la seconde portion de coulissement (226) est en prise coulissante dans la première rainure de coulissement (211) et la seconde rainure de coulissement (212) de l'élément mobile (21) ; et le crochet (224) est en prise avec la seconde portion de coulissement (226) à travers le trou traversant (227), de sorte qu'une extrémité de la première portion de coulissement (225) bute contre la plaque de recouvrement (223).

8. Dispositif de transport de stent (100) selon l'une quelconque des revendications 1 à 2 ou 4 à 7, dans lequel l'ensemble de fixation (1) comporte en outre un élément de guidage (13), l'élément de guidage (13) est agencé à l'opposé de l'élément de fixation (11) avec un espace de réception (14) défini entre l'élément de guidage (13) et l'élément de fixation (11) et l'espace de réception (14) est conçu pour recevoir une portion d'extrémité du stent (200) et dans lequel une extrémité de l'élément de limitation (12) traverse l'élément de guidage (13) et s'étend dans l'espace de réception (14) et traverse la portion d'extrémité du stent (200) et est reliée à l'élément de fixation (11).

9. Dispositif de transport de stent (100) selon la revendication 8, dans lequel le dispositif de transport de stent (100) a la caractéristique k) ou les caractéristiques k) et l) ou les caractéristiques k, l) et m) supplémentaires :
k) l'élément de fixation (11) est doté d'une première surface (111) opposée à l'élément de guidage (13), la première surface (111) est dotée d'au moins un premier trou (112) ; et lorsque l'élément de verrouillage (22) est dans la première position ou la seconde position, une extrémité de l'élément de limitation (12) s'étend dans le premier trou (112) ou en sort lorsqu'elle est entraînée par l'élément mobile (21) ;
l) l'élément de guidage (13) est doté d'au moins un second trou (133) et le second trou (133) est aligné sur le premier trou (112) et l'élément de limitation (12) traverse le second trou (133) et est relié de manière coulissante à l'élément de guidage (13) ;
m) lorsque l'élément de limitation (12) s'éloigne de l'élément de fixation (11) lorsqu'il est entraîné par l'élément mobile (21), une portion d'extrémité de l'élément de limitation (12) est capable de se rétracter dans le second trou (133) et la portion d'extrémité du stent (200) est désolidarisée de l'élément de limitation (12) en raison d'un blocage de l'élément de guidage (13).

10. Dispositif de transport de stent (100) selon l'une quelconque des revendications 1 à 2, 4 à 7 ou 9, dans lequel le dispositif de transport de stent (100) comporte en outre la caractéristique n) ou les caractéristiques n) et o) supplémentaires :
n) un noyau intérieur (3), dans lequel l'élément de fixation (11) et l'élément mobile (21) sont montés autour d'une périphérie extérieure du noyau intérieur (3), l'élément de fixation (11) est fixé par rapport au noyau intérieur (3) et l'élément mobile (21) est relié de manière coulissante au noyau intérieur (3) ;
o) une gaine extérieure (4), dans lequel la gaine extérieure (4) est montée autour de la périphérie extérieure du noyau intérieur (3), une cavité de réception (40) est définie entre la gaine extérieure (4) et le noyau intérieur (3) et le stent (200) est comprimé au sein de la cavité de réception (40).

11. Dispositif de transport de stent (100) selon la revendication 10, dans lequel, lorsque le dispositif de transport de stent (100) a les caractéristiques n) et o), le dispositif de transport de stent (100) a en outre la caractéristique p), q) ou r) supplémentaire :
p) le noyau intérieur (3) et la gaine extérieure (4) sont constitués d'un matériau flexible ;
q) l'élément de limitation (12) est agencé au sein de la cavité de réception (40) et le stent (200) est agencé entre l'élément de limitation (12) et la gaine extérieure (4) ;
r) une extrémité de la gaine extérieure (4) bute contre l'élément de fixation (11), la gaine extérieure (4) est capable de coulisser par rapport au noyau intérieur (3) ; et dans lequel, lorsque la gaine extérieure (4) s'éloigne de l'élément de fixation (11), une portion du stent (200) exposée à partir de la gaine extérieure (4) se déploie sous une action de sa propre tension.

12. Dispositif de transport de stent (100) selon la revendication 11, dans lequel, lorsque le dispositif de transport de stent (100) a la caractéristique r), un espace de rétractation (41) est prévu entre une autre extrémité de la gaine extérieure (4) et l'ensemble de libération (2) ; et dans lequel, lorsque la gaine extérieure (4) s'éloigne de l'élément de fixation (11), l'autre extrémité de la gaine extérieure (4) s'étend dans l'espace de rétractation (41).

13. Dispositif de transport de stent (100) selon la revendication 12, dans lequel le dispositif de transport de stent (100) comporte en outre la caractéristique s) ou les caractéristiques s) et t) supplémentaires :
s) un ensemble de coulissement de gaine extérieure (5), dans lequel l'ensemble de coulissement de gaine extérieure (5) est agencé entre l'ensemble de fixation (1) et l'ensemble de libération (2), l'ensemble de coulissement de gaine extérieure (5) est monté autour d'une périphérie extérieure de la gaine extérieure (4) et l'ensemble de coulissement de gaine extérieure (5) est conçu pour amener la gaine extérieure (4) à s'éloigner ou se rapprocher de l'élément de fixation (11) ;
t) l'ensemble de coulissement de gaine extérieure (5) comporte un deuxième manchon (51) et un ensemble d'entraînement (52) relié de manière filetée au deuxième manchon (51) et le deuxième manchon (51) est monté autour de la périphérie extérieure de la gaine extérieure (4) et relié de manière coulissante à la gaine extérieure (4) ; l'ensemble d'entraînement (52) est monté autour de la périphérie extérieure de la gaine extérieure (4) et relié de manière fixe à la gaine extérieure (4) ; et dans lequel, lorsque l'ensemble d'entraînement (52) tourne par rapport au deuxième manchon (51), l'ensemble d'entraînement (52) s'éloigne progressivement du deuxième manchon (51) et la gaine extérieure (4) entraînée par l'ensemble d'entraînement (52) s'éloigne progressivement de l'élément de fixation (11).

14. Dispositif de transport de stent (100) selon la revendication 13, dans lequel, lorsque le dispositif de transport de stent (100) a les caractéristiques s) et t), l'ensemble d'entraînement (52) comporte un élément de verrouillage (521) et un manchon coulissant (522), une extrémité de l'élément de verrouillage (521) est reliée de manière filetée au deuxième manchon (51), l'élément de verrouillage (521) est relié de manière coulissante à la gaine extérieure (4) et une autre extrémité de l'élément de déverrouillage (521) est reliée de manière détachable au manchon coulissant (522) ; et le manchon coulissant (522) est relié de manière fixe à la gaine extérieure (4) et dans lequel, lorsque l'élément de verrouillage (22) est désolidarisé du manchon coulissant (522), le manchon coulissant (522) est capable d'amener la gaine extérieure (4) à s'éloigner progressivement de l'élément de fixation (11).

15. Dispositif de transport de stent (100) selon la revendication 14, dans lequel le dispositif de transport de stent (100) a la caractéristique u) ou les caractéristiques u) et v) supplémentaires ;
u) l'élément de verrouillage (22) comporte un troisième manchon (523) et un commutateur (524) prévu sur le troisième manchon (523), le troisième manchon (523) est monté autour de la gaine extérieure (4) et peut coulisser par rapport à la gaine extérieure (4), le commutateur (524) est relié de manière coulissante au troisième manchon (523) le long d'une direction radiale du troisième manchon (523) et le commutateur (524) a une portion de bouclage (528) en prise avec le manchon coulissant (522) ; et dans lequel, lorsque le commutateur (524) coulisse vers une position pressée, la portion de bouclage (528) du commutateur (524) est désolidarisée du manchon coulissant (522) ;
v) le commutateur (524) est doté d'une portion de pression (525), d'un élément élastique (526) et d'une base (527) agencés à la suite le long d'une direction radiale du troisième manchon (523), la portion de pression (525) est agencée sur une surface périphérique extérieure du troisième manchon (523), l'élément élastique (526) bute de façon élastique entre la portion de pression (525) et la base (527) avec un espace (529) défini entre la portion de pression (525) et la base (527) ; la portion de bouclage (528) s'étend vers le manchon coulissant (522) à partir d'une extrémité de la portion de pression (525) ; et dans lequel, lorsque la portion de pression (525) bute contre la base (527), le commutateur (524) est dans la position pressée ; tandis que, lorsque le manchon coulissant (522) bute contre le troisième manchon (523) et que le commutateur (524) rebondit à partir de la position pressée sous une action de l'élément élastique (526), la portion de bouclage (528) du commutateur (524) est mise en prise avec le manchon coulissant (522) sous une action de la portion de pression (525).
